# EUROPÄISCHE PATENTSCHRIFT

(11) **EP 0 542 086 B1**
(45) Veröffentlichungstag und Bekanntmachung des Hinweises auf die Patenterteilung: **02.11.1994**
(21) Anmeldenummer: 92118743.1
(22) Anmeldetag: 02.11.1992
(51) Int. Cl.: C07D 213/61

(54) **Verfahren zur Herstellung von N-[(2-Chlor-pyridin-5-yl)methyl]-ethylendiamin**
Process for the preparation of the N-[(2-chloropyridin-5-yl)methyl]-ethylenediamine
Procédé de préparation de la N-[2-chloropyridin-5-yl)méthyl]éthylènediamine

(30) Priorität: 13.11.1991 DE 4137271
(43) Veröffentlichungstag der Anmeldung: 19.05.1993
(73) Patentinhaber: BAYER AG, 51368 Leverkusen (DE)
(72) Erfinder: Lantzsch, Reinhard, Dr., W-5600 Wuppertal (DE)

(56) Entgegenhaltungen:
- EP-A- 0 163 855
- EP-A- 0 192 060
- EP-A- 0 302 389
- FR-A- 2 232 326
- MILOS HUDLICKY ''Reductions in organic chemistry'' 1984 , ELLIS HORWOOD LTD. , CHICHESTER GB
- Journal of Organic Chemistry, 1963, 28, Pages 3259-3261

## Beschreibung

Die Erfindung betrifft ein neues Verfahren zur Herstellung von N-(2-Chlor-pyridin-5-yl-methyl)-ethylendiamin.

Eine reduktive Reaktion von Benzaldehyd mit Anilin in Gegenwart von Natriumborhydrid ist bekannt aus J.Org.Chem. 1963, 28, S. 3259-3261.

Es ist bekannt, daß man N-(2-Chlor-pyridin-5-yl-methyl)-ethylendiamin, ein Zwischenprodukt für Insektizide erhält, wenn man 2-Chlor-5-chlormethyl-pyridin mit Ethylendiamin in Acetonitril umsetzt (vgl. EP-A 163 855, Beispiel 1). Diese Umsetzung verläuft jedoch nicht ausreichend selektiv, was zu einer Minderung der Ausbeute am gewünschten Produkt führt.

In EP-A 163 855 wird auch auf die Möglichkeit hingewiesen, (Pyridylmethyl)-alkylendiaminderivate durch Umsetzung von Pyridinaldehyden oder -ketonen mit Alkylendiaminen in Gegenwart von Natriumborhydrid zu erhalten. Es geht daraus jedoch nichts darüber hervor, daß sich diese Methode besonders gut zur Herstellung von N-(2-Chlorpyridin-5yl-methyl)ethylendiamin eignet.

Es wurde nun ein Verfahren zur Herstellung von N-(2-Chlor-pyridin-5-yl-methyl)-ethylendiamin der Formel (I) 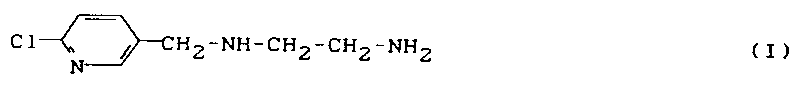
gefunden, welches dadurch gekennzeichnet ist, daß man 6-Chlor-nicotinaldehyd der Formel (II) 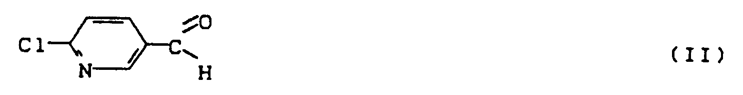
mit 1,2-Ethylendiamin der Formel (III)

H₂N-CH₂-CH₂-NH₂ (III)

und einem Reduktionsmittel in Gegenwart eines Verdünnungsmittels bei Temperaturen zwischen -30°C und +50°C umsetzt.

Überraschenderweise kann N-(2-Chlor-pyridin-5-yl-methyl)-ethylendiamin nach dem erfindungsgemäßen Verfahren auf technisch einfache Weise in hoher Ausbeute und in guter Qualität hergestellt werden. Wie ein Vergleichsversuch ohne Verwendung eines Reduktionsmittels zeigt, ist nämlich mit einer Kondensationsreaktion auch am zweiten Stickstoffatom des Ethylendiamins zu rechnen, die bei der erfindungsgemäßen Verfahrensführung überraschenderweise vermieden wird.

Der Reaktionsablauf beim erfindungsgemäßen Verfahren kann durch das folgende Formelschema skizziert werden: 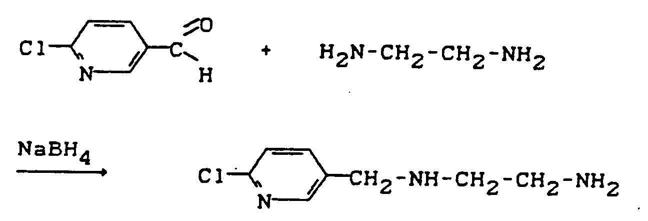
Der beim erfindungsgemäßen Verfahren als Ausgangsstoff zu verwendende 6-Chlor-nicotinaldehyd der Formel (II) ist bereits bekannt (vgl. z.B. EP-A 411 364).

Das weiter als Ausgangsstoff benötigte Ethylendiamin ist eine bekannte Synthesechemikalie.

Das erfindungsgemäße Verfahren wird unter Verwendung eines Reduktionsmittels durchgeführt. Als Reduktionsmittel kommen hierbei vorzugsweise Metallhydridkomplexe in Betracht. Als Beispiele hierfür seien genannt: Lithiumalanat (LiAlH₄), Lithiumboranat (LiBH₄), Natriumboranat (NaBH₄) und Kaliumboranat (KBH₄). Insbesondere Natriumboranat wird beim erfindungsgemäßen Verfahren als bevorzugtes Reduktionsmittel eingesetzt.

Das erfindungsgemäße Verfahren wird unter Verwendung eines Verdünnungsmittels durchgeführt. Vorzugsweise werden polare Lösungsmittel, insbesondere Alkohole, wie Methanol, Ethanol, Propanol, Isopropanol, Butanol, Isobutanol, sec-Butanol und tert-Butanol, verwendet. Methanol wird als Verdünnungsmittel für das erfindungsgemäße Verfahren ganz besonders bevorzugt.

Die Reaktion wird im Temperaturbereich von -30°C bis +50°C durchgeführt. Vorzugsweise arbeitet man bei Temperaturen zwischen -10 °C und +30 °C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens setzt man auf 1 Mol 6-Chlor-nicotinaldehyd der Formel (II) im allgemeinen zwischen 1 und 5 Mol, vorzugsweise zwischen 2 und 4 Mol, Ethylendiamin und zwischen 0,5 und 3 Mol, vorzugsweise zwischen 1,0 und 1,5 Mol, Reduktionsmittel ein.

In einer bevorzugten Ausführungsform des erfindungsgemäßen Verfahrens wird das Ethylendiamin in einem Verdünnungsmittel vorgelegt und eine Lösung des 6-Chlor-nicotinaldehyds langsam dazu gegeben. Nach kurzem Durchrühren der Mischung wird dann das Reduktionsmittel langsam zudosiert und das Reaktionsgemisch wird bis zum Ende der Umsetzung gerührt. Die Aufarbeitung kann nach üblichen Methoden durchgeführt werden (vgl. die Herstellungsbeispiele).

Das nach dem erfindungsgemäßen Verfahren herzustellende N-(2-Chlor-pyridin-5-yl-methyl)-ethylendiamin kann als Zwischenprodukt zur Herstellung von Insektiziden verwendet werden (vgl. EP-A 163 855).

Als Reaktionsschema für die weitere Umsetzung zum Erhalt insektizider Wirkstoffe sei beispielhaft aufgeführt:

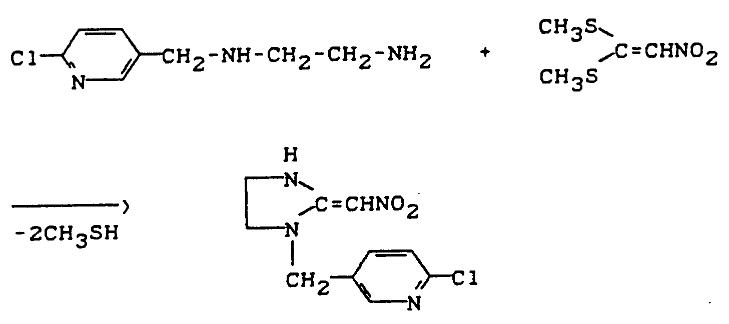

### Beispiel 1

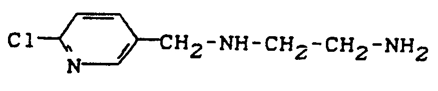

12 g (0,2 Mol) Ethylendiamin in 70 ml Methanol werden vorgelegt. Man tropft 7,1 g (0,05 Mol) 6-Chlor-nicotinaldehyd, gelöst in 45 ml Methanol, bei 0°C zu. Man rührt 1 Stunde bei 0°C nach und gibt dann portionsweise 1,95 g (0,05 Mol) Natriumboranat bei 0°C hinzu. Das Reaktionsgemisch wird 12 Stunden ohne Kühlung nachgerührt, die Reaktionslösung filtriert und das Filtrat im Wasserstrahlvakuum eingeengt. Das eingeengte Filtrat wird mit 50 ml gesättigter Kochsalzlösung versetzt und viermal mit Essigsäureethylester extrahiert. Die vereinigten Extrakte werden mit Magnesiumsulfat getrocknet und im Vakuum eingeengt.

Man erhält 7,4 g N-(2-Chlor-pyridin-5-yl-methyl)-ethylendiamin mit einem Gehalt von 94,2% (nach Gaschromatogramm). Dies entspricht einer Ausbeute von 75% der Theorie.

### Beispiel 2

Die Umsetzung in Beispiel 2 wird wie in Beispiel 1 beschrieben durchgeführt. Die Aufarbeitung wird wie folgt durchgeführt: Man destilliert Methanol und Ethylendiamin-Überschuß im Wasserstrahlvakuum ab, nimmt den Rückstand in Wasser auf und filtriert. Diese wäßrige Lösung enthält nach HPLC-Analyse 8,4 g (90,5% der Theorie) N-(2-Chlor-pyridin-5-yl-methyl)-ethylendiamin.

### Vergleichsbeispiel:

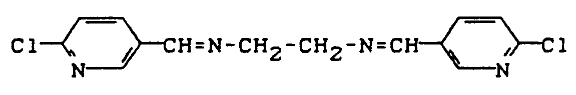

12 g (0,2 Mol) Ethylendiamin in 70 ml Methanol werden vorgelegt. Man tropft 7,1 g (0,05 Mol) 6-Chlor-nicotinaldehyd, gelöst in 45 ml Methanol, bei 0°C zu und rührt 3 Stunden bei 0°C nach. Methanol wird im Vakuum abdestilliert, der Rückstand in ca. 100 ml Essigester aufgenommen und zweimal mit Kochsalzlösung gewaschen. Die organische Phase wird abgetrennt, getrocknet und eingeengt.

Man erhält 5 g N,N'-Bis-(2-chlor-pyridin-5-yl-methylen)-ethylendiamin vom Schmelzpunkt 170°C.

Bei kürzerer Umsetzungszeit enthält das Reaktionsgemisch noch nicht umgesetztes Ausgangsprodukt 6-Chlor-nicotinaldehyd.

## Patentansprüche

1. Verfahren zur Herstellung von N-(2-Chlor-pyridin-5-yl-methyl)-ethylendiamin der Formel (I) 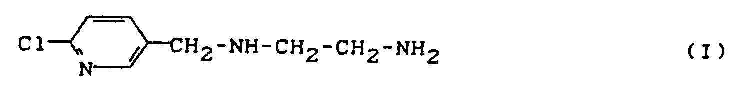 dadurch gekennzeichnet, daß man 6-Chlor-nicotinaldehyd der Formel (II) 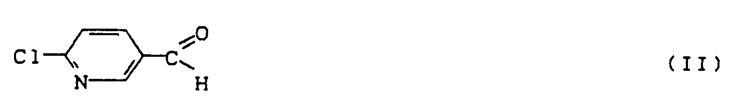 mit 1,2-Ethylendiamin der Formel (III)
H₂N-CH₂-CH₂-NH₂ (III)
und einem Reduktionsmittel in Gegenwart eines Verdünnungsmittels bei Temperaturen zwischen -30°C und +50°C umsetzt.

2. Verfahren gemäß Anspruch 1, dadurch gekennzeichnet, daß man als Reduktionsmittel eine Metallhydridkomplex-Verbindung einsetzt.

3. Verfahren gemäß Anspruch 1 bis 2, dadurch gekennzeichnet, daß man als Reduktionsmittel eine Metallhydridkomplex-Verbindung der Reihe Lithiumalanat, Lithiumboranat, Natriumboranat, Kaliumboranat einsetzt.

4. Verfahren gemäß Anspruch 1 bis 3, dadurch gekennzeichnet, daß man als Reduktionsmittel Natriumboranat einsetzt.

5. Verfahren gemäß Anspruch 1 bis 4, dadurch gekennzeichnet, daß man als Verdünnungsmittel einen Alkohol einsetzt.

6. Verfahren gemäß Anspruch 1 bis 5, dadurch gekennzeichnet, daß man als Verdünnungsmittel Methanol einsetzt.

7. Verfahren gemäß Anspruch 1 bis 6, dadurch gekennzeichnet, daß man bei Temperaturen zwischen -10°C und +30°C arbeitet.

8. Verfahren gemäß Anspruch 1 bis 7, dadurch gekennzeichnet, daß man pro Mol 6-Chlor-nicotinaldehyd der Formel (II) 1 bis 5 Mol 1,2-Ethylendiamin und 0,5 bis 3 Mol Reduktionsmittel einsetzt.

9. Verfahren gemäß Anspruch 1 bis 8, dadurch gekennzeichnet, daß man pro Mol 6-Chlor-nicotinaldehyd der Formel (II) 2 bis 4 Mol 1,2-Ethylendiamin und 1 bis 1,5 Mol Reduktionsmittel einsetzt.

10. Verfahren gemäß Anspruch 1 bis 9, dadurch gekennzeichnet, daß man das 1,2-Ethylendiamin in einem Verdünnungsmittel vorlegt, eine Lösung des 6-Chlor-nicotinaldehyds langsam zugibt, nach kurzem Durchrühren der Mischung das Reduktionsmittel langsam zudosiert, das Reaktionsgemisch bis zum Ende der Umsetzung rührt und das Reaktionsprodukt nach üblichen Methoden aufarbeitet.

## Claims

1. Process for the preparation of N-(2-chloro-pyridin-5-yl-methyl)-ethylenediamine, of the formula (I), 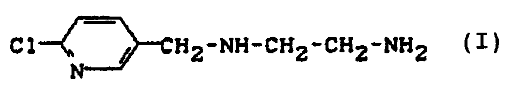 characterized in that 6-chloro-nicotinaldehyde, of the formula (II) 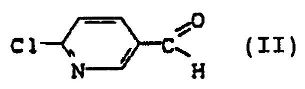 is reacted with 1,2-ethylenediamine of the formula (III)
H₂N-CH₂-CH₂-NH₂ (III)
and a reducing agent in the presence of a diluent at temperatures between -30°C and +50°C.

2. Process according to Claim 1, characterized in that a metal hydride complex compound is employed as the reducing agent.

3. Process according to Claims 1 and 2, characterized in that a metal hydride complex compound from the series comprising lithium aluminium hydride, lithium borohydride, sodium borohydride and potassium borohydride is employed as the reducing agent.

4. Process according to Claims 1 to 3, characterized in that sodium borohydride is employed as the reducing agent.

5. Process according to Claims 1 to 4, characterized in that an alcohol is employed as the diluent.

6. Process according to Claims 1 to 5, characterized in that methanol is employed as the diluent.

7. Process according to Claims 1 to 6, characterized in that the process is carried out at temperatures between -10°C and +30°C.

8. Process according to Claims 1 to 7, characterized in that 1 to 5 mol of 1,2-ethylenediamine and 0.5 to 3 mol of reducing agent are employed per mole of 6-chloro-nicotinaldehyde, of the formula (II).

9. Process according to Claims 1 to 8, characterized in that 2 to 4 mol of 1,2-ethylenediamine and 1 to 1.5 mol of reducing agent are employed per mole of 6-chloro-nicotinaldehyde, of the formula (II).

10. Process according to Claims 1 to 9, characterized in that the 1,2-ethylenediamine is introduced into a diluent, a solution of the 6-chloro-nicotinaldehyde is added slowly, the mixture is stirred briefly and the reducing agent is then slowly metered in, the reaction mixture is stirred until the reaction is complete, and the reaction product is worked up by customary methods.

## Revendications

1. Procédé de préparation de N-(2-chloropyridin-5-yl-méthyl)-éthylènediamine de formule (I) 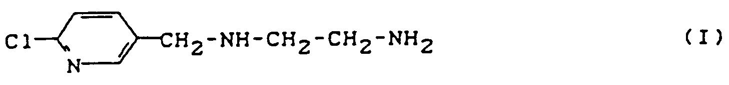 caractérisé en ce qu'on fait réagir du 6-chloronicotinaldéhyde de formule (II) 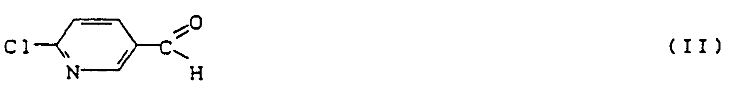 avec de la 1,2-éthylènediamine de formule (III)
H₂N-CH₂-CH₂-NH₂ (III)
et un réducteur en présence d'un milieu de dilution, à des températures comprises entre -30°C et +50°C.

2. Procédé selon la revendication 1, caractérisé en ce qu'on utilise en tant que réducteur un composé complexe d'hydrure métallique.

3. Procédé selon les revendications 1 à 2, caractérisé en ce qu'on utilise en tant que réducteur un composé complexe d'hydrure métallique de la série de l'alanate de lithium, du boranate de lithium, du boranate de sodium, du boranate de potassium.

4. Procédé selon les revendications 1 à 3, caractérisé en ce qu'on utilise en tant que réducteur du boranate de sodium.

5. Procédé selon les revendications 1 à 4, caractérisé en ce qu'on utilise un alcool en tant que milieu de dilution.

6. Procédé selon les revendications 1 à 5, caractérisé en ce qu'on utilise le méthanol en tant que milieu de dilution.

7. Procédé selon les revendications 1 à 6, caractérisé en ce qu'on travaille à des températures comprises entre -10°C et +30°C.

8. Procédé selon les revendications 1 à 7, caractérisé en ce que pour une mole de 6-chloronicotinaldéhyde de formule (II) on utilise 1 à 5 mol de 1,2-éthylènediamine et 0,5 à 3 mol de réducteur.

9. Procédé selon les revendications 1 à 8, caractérisé en ce que pour une mole de 6-chloronicotinaldéhyde de formule (II) on utilise 2 à 4 mol de 1,2-éthylènediamine et 1 à 1,5 mol de réducteur.

10. Procédé selon les revendications 1 à 9, caractérisé en ce qu'on introduit au préalable la 1,2-éthylènediamine dans un milieu de dilution, qu'on ajoute lentement une solution du 6-chloronicotinaldéhyde, qu'après une agitation vigoureuse brève du mélange on effectue une addition dosée lente du réducteur, qu'on agite le mélange réactionnel jusqu'à la fin de la réaction et qu'on traite le produit réactionnel selon des méthodes usuelles.
